**Europäisches Patentamt**

**European Patent Office**

**Office européen des brevets**

(19)

(11) Veröffentlichungsnummer: **0 201 667**
**B1**

(12) **EUROPÄISCHE PATENTSCHRIFT**

(45) Veröffentlichungstag der Patentschrift:
19.04.89

(51) Int. Cl.⁴: **A 61 F 2/08**

(21) Anmeldenummer: **86101642.6**

(22) Anmeldetag: **06.02.86**

(54) Künstliches Band aus textilen Schlauchgebilden.

(30) Priorität: **03.05.85  CH 1891/85**

(43) Veröffentlichungstag der Anmeldung:
20.11.86 Patentblatt 86/47

(45) Bekanntmachung des Hinweises auf die Patenterteilung:
19.04.89 Patentblatt 89/16

(84) Benannte Vertragsstaaten:
AT DE FR GB IT

(56) Entgegenhaltungen:
EP-A-0 126 520
FR-A-2 135 825
FR-A-2 395 012
US-A-3 176 316
US-A-3 882 551
US-A-4 255 820
US-A-4 301 551

(73) Patentinhaber: **GEBRÜDER SULZER
AKTIENGESELLSCHAFT**, Zürcherstrasse 9, CH-
8401 Winterthur (CH)
Patentinhaber: **Protek AG**, Stadtbachstrasse 64, CH-
3001 Bern (CH)

(72) Erfinder: **Mansat, Christian, Dr.- med., Route de
Gaure, F-31130 Balma (FR)**

(74) Vertreter: **Dipl.- Ing. H. Marsch Dipl.- Ing. K.
Sparing Dipl.- Phys.Dr. W.H. Röhl
Patentanwälte, Rethelstrasse 123, D-4000
Düsseldorf 1 (DE)**

## Beschreibung

Die Erfindung betrifft ein künstliches Band aus textilen Schlauchgebilden, die einen in axialer Richtung elastisch dehnbaren Strang bilden, wobei in axialer Richtung Bereiche mit unterschiedlicher Flexibilität und unterschiedlicher elastischer Längsdehnbarkeit vorhanden sind; unter "textilen Schlauchgebilden" werden dabei aus multifilen Fäden oder Monofilamenten, beispielsweise Drähten, hergestellte "Hohlzylinder" verstanden, wobei alle für die Verarbeitung von Fäden bekannten Verfahren, wie Flechten, Weben, Stricken, Wirken usw. benutzt werden können, die einen Mindestwert für die elastische Längsdehnbarkeit des Bandes zulassen.

Aus der A-2 395 012 ist ein Sehnenersatz bekannt, der aus geflochtenen Kunststoff-Fäden besteht. Dieses Geflecht ist von einer abnehmbaren Hülle umschlossen, die mit pyrolytischem Kohlenstoff beschichtet ist. Durch Änderungen des Flechtwinkels ist die Biegsamkeit oder Flexibilität und die Längsdehnbarkeit dieser Kunstsehne in einem Endbereich verändert, in dem eine Verbindung mit der zu ersetzenden teilweise fehlenden natürlichen Sehne erfolgt. In ihrem eigentlichen Funktionsbereich, d. h. ausserhalb der der Verbindung zur natürlichen Sehne dienenden Endbereiche, weist die bekannte Kunstsehnekonstante Querschnitte und einheitliche Flexibilität sowie Längsdehnbarkeit auf; ihre Längsdehnbarkeit ist dabei eng begrenzt.

Eine im grundsätzlichen Aufbau ähnliche künstliche Sehne aus Kunstfasern, die ebenfalls zu einem Schlauch geflochten oder gewoben sind, ist in der US-A-3 176 316 beschrieben. An den Enden ist auch diese Sehne, die in ihrem Funktionsbereich einen relativ kompakten, in Längsrichtung unelastischen "Schaft" mindestens nahezu konstanten Durchmessers bildet, ein relativ lockerer hohler Schlauch, der über den Stumpf der zu ersetzenden natürlichen Sehne gezogen wird.

Es hat sich gezeigt, dass für künstliche Bänder, z. B. für Kreuz- oder Seitenbänder am Kniegelenk, die Flexibilität und die Längsdehnbarkeit der bekannten Konstruktionen ungenügend sind. Aufgabe der Erfindung ist es daher, ein künstliches Band mit hoher Längsdehnbarkeit und hoher Flexibilität sowie relativ grosser Torsionssteife im Funktionsbereich und geringen Werten für die Längsdehnbarkeit und die Flexibilität im Verankerungsbereich zu schaffen. Diese Aufgabe wird mit der Erfindung dadurch gelöst, dass der Strang aus einer Vielzahl konzentrischer Schläuche gebildet ist, deren Anzahl pro Querschnitt in allen Bereichen konstant ist, und dass ferner der Durchmesser des flexiblen Bereichs das 1,2- bis 2-fache desjenigen des steifen Bereichs ist.

Die relativ geringe Durchmesservergrösserung bewirkt bei textilen Schlauchgebilden, die kernlos aus einer Vielzahl von konzentrischen "Lagen" aufgebaut sind, eine Vervielfachung der Flexibilität und der elastischen Längsdehnbarkeit; der für das neue Band erforderliche Mindestwert für die elastische Längsdehnbarkeit beträgt im flexiblen Bereich, beispielsweise bei Zugbelastungen von 50 N/mm², 5 - 25 %.

Eine besonders vorteilhafte Ausführungsform des neuen Bandes erhält man, wenn der Strang aus geflochtenen Schläuchen besteht, deren Flechtwinkel gegen die Achsrichtung in den Bereichen unterschiedlicher Flexiblität verschieden, jedoch innerhalb eines Bereichs für alle Schläuche untereinander gleich sind. Die Schläuche können aus multifilen oder monofilen Fäden hergestellt sein, wobei der Begriff "monofiler Faden" auch metallische Drähte einschliesst; weiterhin können als Fäden Naturfasern, z. B. Seide, oder Kunstfasern, beispielsweise Polyester, Polyäthylen oder auch bekannte resorbierbare Materialien verwendet werden. Zur Herstellung eines Stranges werden eine Vielzahl von konzentrischen Schläuchen oder Lagen, für ein Kreuzband beispielsweise 20 - 30 Schläuche, übereinander "geschichtet".

Für die Herstellung von geflochtenen Schläuchen haben sich besonders gezwirnte Fäden aus einer Vielzahl, z. B. 60, Monofilamenten bewährt, die beispielsweise aus Polyester bestehen und Durchmesser von 0,01 bis 0,03 mm haben. Mit Vorteil kann der äusserste Schlauch - um beispielsweise zur Erhöhung der mechanischen Festigkeit und/oder zur Erzeugung einer relativ dichten und glatten Oberfläche ein möglichst dichtes Geflecht zu erhalten - aus Fäden mit einer erhöhten, beispielsweise einer verdoppelten, Anzahl an Monofilamenten gefertigt sein.

Für den Ersatz eines Kreuzbandes in einem Kniegelenk hat es sich als günstig erwiesen, wenn an einen Bereich hoher Flexibilität beidseits Bereiche geringer Flexibilität anschliessen, wobei der Durchmesser des von Zugspannungen entlasteten Stranges im Bereich hoher Flexiblität 8 - 12 mm betragen kann. Als vorteilhaftes Kriterium für die elastische Längsdehnbarkeit im flexiblen Bereich hat es sich erwiesen, wenn diese elastische Längsdehnbarkeit derart ist, dass bei einer Zugbelastung von 500 N die Durchmesser beider Bereiche mindestens annähernd gleich sind.

Mit dem neuen Band ergibt sich eine sehr einfache Implantationstechnik, die am Beispiel eines Ersatzes des vorderen Kreuzbandes beschrieben sei:

Wie bereits erwähnt, besteht eine Kreuzband-Prothese mit Vorteil aus einem flexiblen Mittelteil, an den sich zu beiden Seiten steife Endbereiche anschliessen. Mit diesen wird das Band aussen einerseits auf dem Schienbein und andererseits auf dem Femur- beispielsweise mit einer Spange oder Agraffe, die in den Knochen eingeschlagen wird - durch Festklemmen gehalten, wobei nach dem Einschlagen der Agraffe das freie Ende der Prothese umgebogen und in einer Bohrung im Knochen versenkt wird. Nach einer Fixierung der Prothese auf dem Tibiaknochen wird der flexible Bereich durch eine Bohrung im Tibiaknochen hindurchgeführt, deren Durchmesser kleiner ist als der Durchmesser des zugbelastungsfreien flexiblen Bereichs; aufgrund des Zuges und infolge der elastischen Längsdehnbarkeit wird dabei das künstliche Band in seinem Durchmesser verkleinert und kann so ohne Schwierigkeiten durch die Bohrung gezogen werden. Beim Nachlassen der Zugspannung "quillt" es dann auf und hat dadurch in der Bohrung innigen Kontakt mit dem Knochen, wodurch das An- und Einwachsen von Gewebe gefördert wird. Nach dem Austritt aus der Tibiabohrung verläuft das künstliche Band zwischen den Kondylen hindurch, umschlingt teilweise die laterale Kondyle im dorsalen Bereich und wird lateral aussen am Femur in der beschriebenen Weise festgeklemmt.

Im folgenden wird die Erfindung anhand eines Ausführungsbeispiels im Zusammenhang mit der Zeichnung näher erläutert.

Fig. 1 ist eine schematische Ansicht eines Bandes nach der Erfindung, etwa in natürlicher Grösse;

Fig. 2 gibt vergrössert den Schnitt II - II von Fig. 1 wieder, während

Fig. 3, ebenfalls etwa in natürlicher Grösse, in einem Schnitt durch einen Knochen eine Möglichkeit für die Fixierung des Bandes aufzeigt.

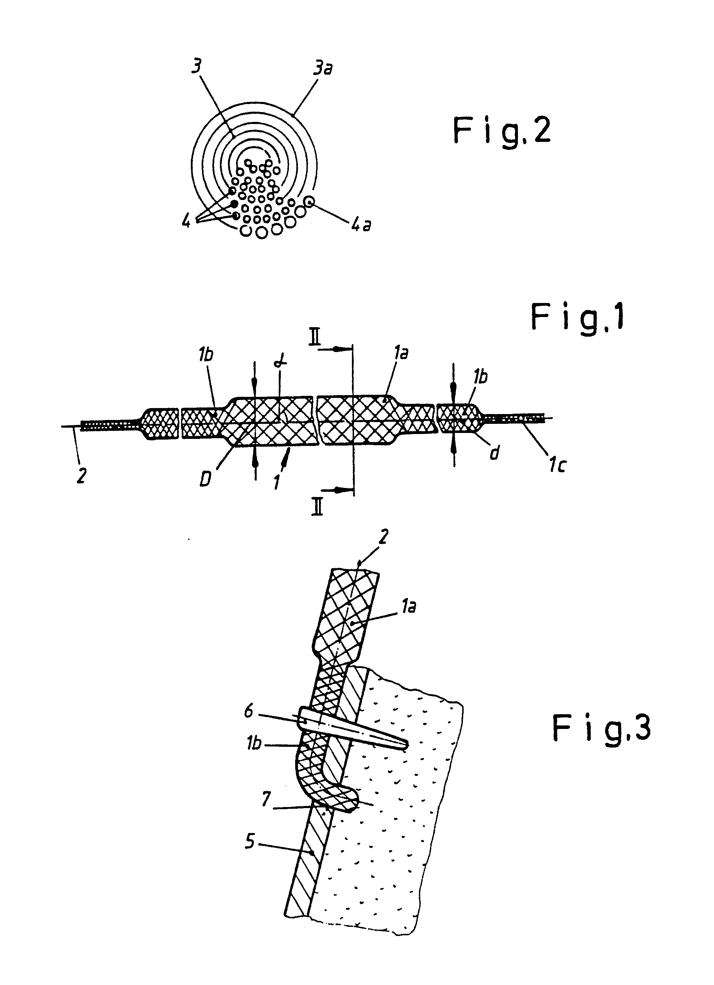

Das beispielsweise als Kreuzbandersatz dienende Band 1 hat einen zentralen flexiblen Bereich 1a, dessen Durchmesser mit D bezeichnet ist; an den Bereich 1a schliessen links und rechts Bereiche 1b an, die eine relativ geringe Flexibilität haben und deren Durchmesser mit d bezeichnet ist. Bei dem etwa in natürlicher Grösse wiedergegebenen Band 1 nach Fig. 1, das für den Ersatz eines Kreuzbandes bestimmt ist, betragen der Durchmesser im flexiblen Bereich D etwa 12 mm und derjenige d im steifen Bereich etwa 7 mm, was ein Verhältnis von etwa 1,7 ergibt.

An den Enden sind einige wenige oder nur der äusserste Schlauch als dünne Einfädelabschnitte 1c ausgebildet, die unter Umständen durch eine Einlage oder ein Tränken in einer härtenden Masse versteift sind.

Die unterschiedliche Längsdehnbarkeit und die unterschiedliche Flexibilität der einzelnen Bereiche 1a und 1b werden beim Aufbau des Bandes 1 aus geflochtenen Schläuchen 3 durch unterschiedliche Flechtwinkel gegen die Bandachse 2 erreicht. Dabei ist die Flexibilität umso grösser je flacher der Flechtwinkel $\alpha$ gegen die Bandachse 2 geneigt ist.

Wie Fig. 2 zeigt, ist das künstliche Band 1 - wegen der notwendigen elastischen Längsdehnbarkeit, die bei einer Belastung mit 50 $N/mm^2$ mindestens 5 - 25 % betragen soll, kernlos - aus einer Vielzahl konzentrischer Schläuche 3 geflochten; die einzelnen Fäden der Schläuche 3 sind in dem gezeigten Beispiel aus einer Vielzahl von Monofilamenten zu einem multifilen Faden verzwirnt, was nicht ausdrücklich gezeigt ist. Um im flexiblen Bereich 1a eine relativ dichte und glatte Oberfläche des Bandes 1 zu erhalten, sind die Fäden 4a des äussersten Schlauches 3a dicker, d. h. sie bestehen beispielsweise aus der doppelten Anzahl an Monofilamenten wie die Fäden 4.

Die Fixierung des künstlichen Bandes 1 im Knochen 5 (Fig. 3) erfolgt z. B. im einfachsten Fall durch eine Spange oder Agraffe 6, die in den Knochen 5 eingeschlagen wird und das Band 1 mit seinem steifen Bereich 1b auf dem Knochen 5 festklemmt. Wenige Zentimeter ausserhalb der Agraffe 6 wird der Bereich 1b mit einem scharfen Schneidwerkzeug, beispielsweise mit einem Skalpell, abgeschnitten und in eine zuvor gesetzte Bohrung 7 im Knochen 5 versenkt.

**Patentansprüche**

1. Künstliches Band (1) aus textilen Schlauchgebilden (3), die einen in axialer Richtung (2) elastisch dehnbaren Strang bilden, wobei in axialer Richtung Bereiche (1a, 1b) mit unterschiedlicher Flexibilität und unterschiedlicher elastischer Längsdehnbarkeit vorhanden sind, dadurch gekennzeichnet, dass der Strang aus einer Vielzahl konzentrischer Schläuche (3, 3a) gebildet ist, deren Anzahl pro Querschnitt in allen Bereichen (1a, 1b) konstant ist, und dass der Durchmesser (D) des flexiblen Bereichs (1a) das 1,2- bis 2-fache desjenigen (d) des steifen Bereichs (1b) ist.

2. Band nach Anspruch 1, dadurch gekennzeichnet, dass der Strang aus geflochtenen Schläuchen (3, 3a) besteht, deren Flechtwinkel ($\alpha$) gegen die Achsrichtung (2) in den Bereichen (1a, 1b) unterschiedlicher Flexiblität verschieden, jedoch innerhalb eines Bereichs (1a, 1b) für alle Schläuche (3, 3a) untereinander gleich sind.

3. Band nach Anspruch 1, dadurch gekennzeichnet, dass die Fäden (4, 4a) zur Herstellung der Schläuche (3, 3a) aus einer Vielzahl von Monofilamenten bestehen.

4. Band nach Anspruch 3, dadurch gekennzeichnet, dass die Fäden (4, 4a) gezwirnt sind.

5. Band nach Anspruch 1, dadurch gekennzeichnet, dass die Querschnitte der zu einem Schlauch verarbeiteten Fäden (4, 4a) beim äussersten Schlauch (3a) grösser sind als bei den übrigen Schläuchen (3).

6. Band nach Anspruch 1, dadurch gekennzeichnet, dass an einen Bereich (1a) hoher Flexibilität beidseits Bereiche (1b) geringer Flexibilität anschliessen.

7. Band nach Anspruch 6, dadurch gekennzeichnet, dass der Durchmesser (D) des von Zugspannungen entlasteten Stranges im Bereich (1a) hoher Flexibilität für einen Kreuzbandersatz 8 - 12 mm beträgt.

8. Band nach Anspruch 1, dadurch gekennzeichnet, dass die elastische Längsdehnbarkeit des flexiblen Bereichs (1a) derart ist, dass bei einer Zugbelastung von 500 N die Durchmesser (D, d) beider Bereiche (1a, 1b) mindestens annähernd gleich sind.

## Claims

1. A prosthetic tendon or ligament or the like (1) made of textile sleeve-like structures (3) having a portion stretchable in the axial direction (2), zones (1a, 1b) of differing flexibility and differing longitudinal stretch being present in the axial direction, characterised in that the axially stretchable portion is embodied by a number of concentric sleeves (3, 3a) whose number per cross-section is constant in all the zones (1a, 1b), and the diameter (D) of the flexible zone (1a) is from 1.2 times to twice the diameter (d) of the rigid zone (1b).

2. A prosthetic tendon or ligament or the like according to claim 2, characterised in that the axially stretchable portion consists of braided sleeves (3, 3a) whose braiding angles ($\alpha$) relatively to the axial direction (2) differ in the zones (1a, 1b) of differing flexibility but are identical to one another in any one zone (1a, 1b) for all the sleeves (3, 3a).

3. A prosthetic tendon or ligament or the like according to claim 1, characterised in that the yarns (4, 4a) from which the sleeves (3, 3a) are made comprise a number of monofilaments.

4. A prosthetic tendon or ligament or the like according to claim 3, characterised in that the yarns (4, 4a) are twisted.

5. A prosthetic tendon or ligament or the like according to claim 1, characterised in that the cross-sections of the yarns (4, 4a) worked up into a sleeve are greater in the outermost sleeve (3a) than in the other sleeves (3).

6. A prosthetic tendon or ligament or the like according to claim 1, characterised in that a high-flexibility zone (1a) merges into low-flexibility zones (1b) on both sides.

7. A prosthetic tendon or ligament or the like according to claim 6, characterised in that in the absence of tension the diameter (D) of the portion in the high-flexibility zone (1a) is from 8 to 12 mm for a prosthetic transverse ligament for a knee.

8. A prosthetic tendon or ligament or the like according to claim 1, characterised in that the longitudinal stretch of the flexible zone (1a) is such that the diameters (D, d) of the two zones (1a, 1b) are at least substantially equal at a tension of 500 N.

## Revendications

1. Ligament synthétique (1) en des structures de flexibles textiles (3) qui forment écheveau extensible élastiquement dans le sens axial (2), des régions (1a, 1b) à flexibilité différente et à extensibilité longitudinale élastique différente étant présentes dans le sens axial, caractérisé par le fait que l'écheveau est formé d'un grand nombre de flexibles concentriques (3, 3a) dont le nombre par section est constant dans toutes les régions (1a, 1b) et par le fait que le diamètre (D) de la région flexible (1a) représente de 1,2 à 2 fois celui (d) de la région rigide (1b).

2. Ligament selon la revendication 1, caractérisé par le fait que l'écheveau est constitué par des flexibles tressés (3, 3a) dont les angles de tressage par rapport à la direction axiale (2), sont différents dans les régions (1a, 1b) à flexibilité différente, mais cependant mutuellement identiques, pour tous les flexibles (3, 3a) à intérieur d'une région (1a, 1b).

3. Ligament selon la revendication 1, caractérisé par le fait que les fils (4, 4a) pour produire les flexibles (3, 3a) sont constitués d'un grand nombre de monofilaments.

4. Ligament selon la revendication 3, caractérisé par le fait que les fils (4, 4a) sont soumis à retordage.

5. Ligament selon la revendication 1, caractérisé par le fait que les sections des fils (4, 4a) travaillés pour former un flexible sont plus grandes, dans le flexible externe extrême (3a), que dans les autres flexibles (3).

6. Ligament selon la revendication 1, caractérisé par le fait que des régions (1b) à faible flexibilité sont attenantes, de part et d'autre, à une région (1a) à flexibilité élevée.

7. Ligament selon la revendication 6, caractérisé par le fait que, pour un ligament croisé, le diamètre (D) de l'écheveau soulagé de contraintes de traction est compris entre 8 et 12 mm dans la région (1a) à flexibilité élevée.

8. Ligament selon la revendication 1, caractérisé par le fait que l'extensibilité longitudinale élastique de la région flexible (1a) est telle que les diamètres (D, d) des deux régions (1a, 1b) soient au moins approximativement identiques en présence d'une contrainte de traction de 500 N.

Fig.2

Fig.1

Fig.3